# EUROPEAN PATENT SPECIFICATION

(11) **EP 1 508 797 B1**
(45) Date of publication and mention of the grant of the patent: **17.12.2008**
(21) Application number: 04019421.9
(22) Date of filing: 16.08.2004
(51) Int. Cl.: G01N 21/89, G01N 33/36, B65H 63/06

(54) **Detecting device for foreign material in yarn**
Detektionsvorrichtung für Fremdstoffe in Garnen
Appareil pour la détection des matières étrangères dans des fils

(30) Priority: 21.08.2003 JP 2003297008
(43) Date of publication of application: 23.02.2005
(73) Proprietor: MURATA KIKAI KABUSHIKI KAISHA, Minami-ku Kyoto-shi Kyoto 601 (JP)
(72) Inventor: Nakade, Kazuhiko, Kyoto-shi Kyoto (JP); Yoshikawa, Keiji, Yokohama-shi Kanagawa (JP)
(74) Representative: Liedl, Christine

(56) References cited:
- EP-A- 1 229 323
- US-A- 4 739 176
- US-A- 5 138 151
- US-A- 5 383 776
- US-A- 5 414 520
- US-A- 5 499 794

## Description

### Field of the Invention

The present invention relates to a detecting device for foreign material that detects foreign material mixed into a spun yarn.

### Background of the Invention

In a spinning machine that aggregates fibers such as cotton together and twists this aggregate to finish a yarn, a detecting device is provided to detect a defect in the yarn, running from a spinning section to a winding bobbin. When a defect occurs in the yarn, the detecting device detects the defect to cut the yarn. The defective part is then removed and then the yarn continues to be finished by, for example, twisting the yarns obtained by the cutting to connect them together.

A known device that detects a defect in the yarn as described above irradiates the running yarn with light and receives light transmitted through the yarn to determine its thickness. The device also receives light reflected by the yarn to detect the mixture of foreign fibers on the basis of the quantity of light received and the determined thickness of the yarn. Such a detecting device is disclosed, for example, in the Japanese Patent Publication No. 3189123.

However, the measurement of the yarn thickness is essential to such a conventional device detecting foreign material in the yarn. Detecting means for this purpose must thus be provided. This complicates the configuration of the foreign material detecting device. Further, the device may fail in detection for a yarn that has been flattened by the mixture of foreign material.

US 4,739,176 discloses a method of monitoring for contaminants in highly elongate textile product such as yarn includes diffusely, applying light to the yarn and monitoring light reflected by the yarn. The yarn is drawn past a background therefore and light is diffusely applied to both the yarn and said background. The background is arranged so that the total amount of monitored light reflected from the yarn and the background is substantially independent of the dimensions and density of the yarn and of the distribution of constituents in the yarn. In this way, a prescribed change in the reflected light intensity indicates a selected contaminant or range of contaminants in the yarn. The background is conveniently provided by an elongate channel so as to be close behind and to each side of the yearn.

EP 1229323 discloses a method of detecting extraneous fibres and/or impurities in a yarn or in another linear fibre textile formation on an operating unit of a textile machine producing or processing yarn or another linear fibre textile formation. In this method, the colour picture of at least a part of the surface of the moving yarn or of another linear fibre textile formation is at predetermined time intervals sensed (registered) and subject to a colour analysis aimed at detecting a spot of different colour in the colour picture of the yarn or of another linear fibre textile formation representing a chromatic aberration due to the presence of an extraneous fibre or of impurities. The device for carrying out the method comprises a radiation source and a sensor of the radiation reflected from the yarn or another linear fibre textile formation whose output is connected with an evaluating device. The radiation sensor consists of at least one row of colour-sensitive sensing elements.

US 5,414,520 discloses a process and device for detecting impurities in a textile test material in which the test sample (G) is illuminated at at least two points (S1, S2) and the reflection from the test sample (G) and also the diameter of the sample or the change therein are measured by receivers (E1, E2). The measurement signals thus obtained are linked together and the signal resulting from this process is examined for differences from a predetermined value. If a difference is detected, there is an impurity in the test sample (G). For use in combination with an electronic yarn clearer for the detection of foreign fibers in yarns.

It is an object of the present invention to solve the above problem by making it possible to accurately and stably detect foreign material mixed into a yarn using a simple configuration.

### Summary of the Invention

The present invention provides a detecting device for foreign material in a yarn according to claim 1.

The detecting device for foreign material in a yarn according to the present invention irradiates the running yarn with light from different angles and allows the at least three appropriately arranged light receiving means to receive light reflected by the yarn. The device thus detects foreign material mixed into the yarn on the basis of the quantity of light reflected. Consequently, the device can reliably detect that the yarn has been flattened by the presence of heterogeneous material such as a polypropylene film. The present invention therefore improves the accuracy with which foreign material is detected. The present invention also requires only the processing of the quantity of light reflected, thus making it possible to simplify the configuration of the detecting device for foreign material in the yarn.

### Brief Description of the Drawings

Figure 1 is a block circuit diagram of a detecting device for foreign material in a yarn according to the present invention.
Figure 2 is a timing chart of a block circuit shown in Figure 1.
Figure 3 is a diagram illustrating operations of the block shown in Figure 1.
Figure 4 is a diagram showing a configuration of a detecting section (sensor head) according to Embodiment 1.
Figure 5 is a diagram showing another configuration of the detecting section according to Embodiment 1.

### Detailed Description of the Preferred Embodiments

The object to accurately and stably detect foreign material mixed into a yarn using a simple configuration has been accomplished by providing a plurality of floodlighting means and at least three light receiving means for receiving that part of light applied by the floodlighting means which is reflected by the yarn.

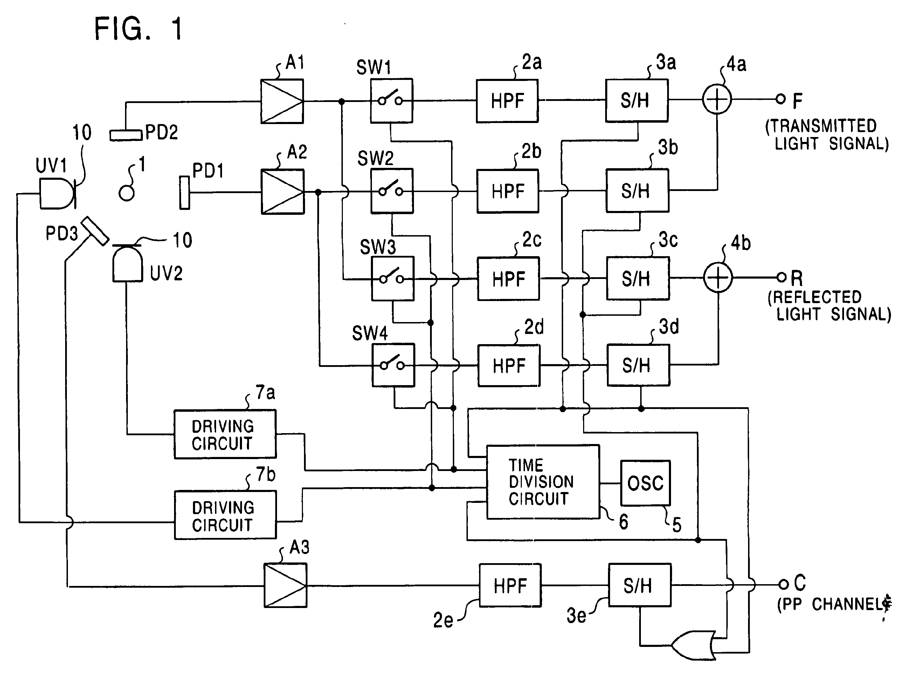

In Figure 1, 1 is a yarn, and UV1, UV2 are light sources, in the present embodiment, light emitting elements such as LEDs which generates ultraviolet rays. PD1, PD2, PD3 are light receiving elements, A1, A2, A3 are amplifiers, and SW1 to SW4 are switching elements. Further, 2a to 2e are high pass filter circuits, 3a to 3e are sample-and-hold circuits, and 4a, 4b are adders. Furthermore, 5 is a transmitter, and 6 is a time division circuit, and 7a, 7b are light source driving circuits, and 10 is a diffuser plate.

The light sources UV1, UV2 apply light to the yarn 1 from two different directions. The light receiving element PD1 receives light applied by the light source UV1 and transmitted through the yarn 1 and light applied by the light source UV1 and reflected by the yarn 1. The light receiving element PD2 receives light applied by the light source UV2 and transmitted through the yarn 1 and light applied by the light source UV1 and reflected by the yarn 1. The light receiving element PD3 receives light applied by the light sources UV1, UV2 and reflected by the yarn 1.

The time division circuit 6 divides an output pulse from the transmitter 5 to simultaneously drive the group of the light source driving circuit 7a, the switching elements SW1, SW4, and the sample-and-hold circuits 3a, 3d. The time division circuit 6 simultaneously drives the group of the light source driving circuit 7b, the switching elements SW2, SW3, and the sample-and-hold circuits 3b, 3c. The time division circuit 6 alternately drives the first group with the second group. The time division circuit 6 also drives the sample-and-hold circuit 3e with a full output pulse.

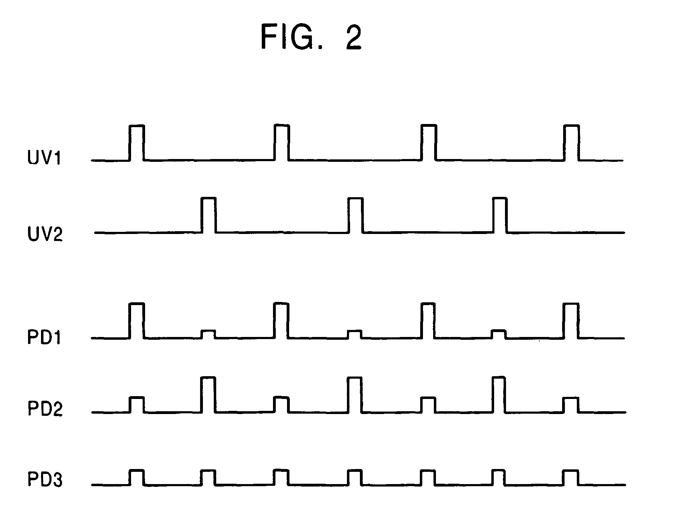

The light source driving circuit 7a is driven. Then, the light source UV2 generates and applies an ultraviolet ray to the yarn 1 while diffusing it via the diffuser plate 10, as shown in Figure 2. The light receiving element PD2 receives a part of the ultraviolet ray which is transmitted through the yarn 1 to output a signal corresponding to the quantity of light received. The light receiving elements PD1, PD3 receive a part of the ultraviolet ray which is reflected by the yarn 1 to output a signal corresponding to the quantity of light received. At this time, the switching elements SW1, SW4 are in a closed state (the switching elements SW2, SW3 are open). The output signal based on the quantity of transmitted light received by the light receiving element PD2 passes through the high pass filter circuit 2a and is then held in the sample-and-hold circuit 3a. The output signal based on the quantity of reflected light received by the light receiving element PD1 passes through the high pass filter circuit 2d and is then held in the sample-and-hold circuit 3d. The output signal based on the quantity of reflected light received by the light receiving element PD3 passes through the high pass filter circuit 2e and is then held in the sample-and-hold circuit 3e.

Then, the light source driving circuit 7b is driven. Accordingly, as shown in Figure 2, the light source UV1 generates and applies an ultraviolet ray to the yarn 1. The light receiving element PD1 receives a part of the ultraviolet ray which is transmitted through the yarn 1 to output a signal corresponding to the quantity of light received. The light receiving elements PD2, PD3 receive a part of the ultraviolet ray which is reflected by the yarn 1 to output a signal corresponding to the quantity of light received. At this time, the switching elements SW2, SW3 are in a closed state (the switching elements SW1, SW4 are open). The output signal based on the quantity of transmitted light received by the light receiving element PD1 passes through the high pass filter circuit 2b and is then held in the sample-and-hold circuit 3b. The output signal based on the quantity of reflected light received by the light receiving element PD2 passes through the high pass filter circuit 2c and is then held in the sample-and-hold circuit 3c. The output signal based on the quantity of reflected light received by the light receiving element PD3 passes through the high pass filter circuit 2e and is then held in the sample-and-hold circuit 3e.

The adder 4a adds the signals held in the sample-and-hold circuits 3a, 3b together and outputs the result as a transmitted light signal F. The adder 4b adds the signals held in the sample-and-hold circuits 3c, 3d together and outputs the result as a reflected light signal R. The signal held in the sample-and-hold circuit 3e is outputted as a PP channel C. The transmitted light signal F indicates the thickness of the yarn 1. The reflected light signal R indicates whether or not foreign material is mixed into the yarn 1. The PP channel reflected light signal C complements the reflected light signal R if a polypropylene film used as a packing material to pack cotton fibers used as a material for spun yarns is mixed into the yarn during a mixing and blowing process and fails to be twisted, thus flattening the yarn; in this case, the reflected light signal fails to indicate the presence of the foreign material depending on the position of the flattened part.

That is, floodlighting means is composed of the light sources UV1, UV2 and the light diffuser plate 10. Reflected light receiving means is composed of a circuit composing the light receiving elements PD1, PD2, PD3, the amplifiers A1, A2, A3, the switching elements SW3, SW4, the high pass filter circuits 2c, 2d, 2e, the sample-and-hold circuits 3c, 3d, 3e, and the adder 4b.

By driving the light sources UV1, UV2, the switching elements, and the sample-and-hold circuits synchronously with an output pulse from the time division circuit 6, it is possible to prevent the adverse effects of an externally invading light or the other light source.

Moreover, the mixture of foreign material into the yarn 1 can be monitored from two different directions to more reliably detect foreign material.

Further, the time division circuit 6 allows the input signals from the two directions to be alternately inputted to enable foreign material to be generally continuously monitored.

Furthermore, the time division circuit 6 allows the input signals from the two directions to be alternately inputted to enable the inputting of not only the reflected light but also the transmitted light.

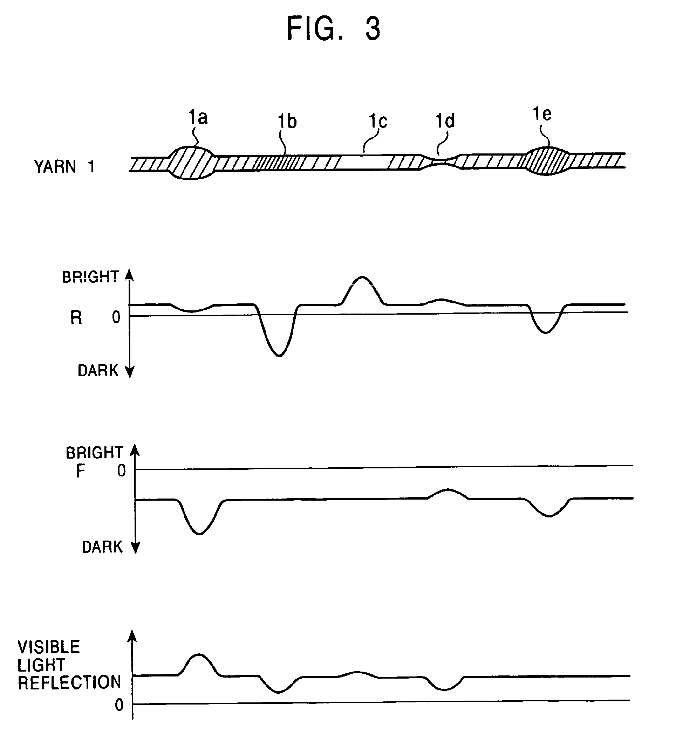

With the above configuration, the yarn 1 is assumed to have a thick portion 1a, a portion 1b into which dark foreign material has been mixed, a portion 1c into which bright foreign material has been mixed, a thin portion 1d, and a portion 1e into which thick and dark foreign material has been mixed, as shown in Figure 3. Then, as shown at R in Figure 3, the reflected light signal R and the PP channel reflected light signal C vary insignificantly in the thick portion 1a and thin portion 1d, and significantly in the portions 1b, 1c, 1e into which foreign material has been mixed. Thus, the mixture of foreign material can be detected regardless of the thickness of the yarn 1.

As shown at F in Figure 3, the transmitted light signal F varies in proportion to the thickness in the thick portion 1a, the thin portion 1d, and the thick portion 1e into which foreign material has been mixed. No change occurs in the thickness unvarying portions 1b, 1c even with the mixture of the foreign material. In the thickness varying portion, the transmitted light signal F varies in proportion to the thickness. This makes it possible to detect a defect in the thickness of the yarn regardless of the mixture of foreign material.

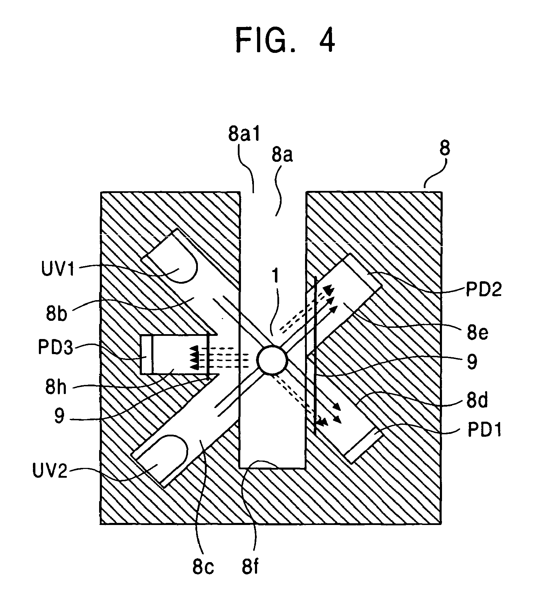

Figure 4 shows a specific example of arrangement of the light sources UV1, UV2, which generate ultraviolet rays, and the light receiving elements PD1, PD2, PD3 which receive the ultraviolet rays from the light sources UV1, UV2. The light sources and the light receiving elements are housed in one case 8.

The case 8 comprises a passage 8a used as a passage space through which the yarn 1 is passed, an opening 8a1 through which the yarn 1 can be inserted into the passage 8a, a hollow chamber 8b in which the light source UV1 is installed, a hollow chamber 8c in which the light source UV2 is installed, a hollow chamber 8d in which the light receiving element PD1 is installed, a hollow chamber 8e in which the light receiving element PD2 is installed, and a hollow chamber 8h in which the light receiving element PD3 is installed.

The hollow chambers 8b, 8c, 8d, 8e, 8h are open toward the yarn 1. A visible light cut filter 9 is provided at each of the openings in the hollow chambers 8d, 8e, 8h. Diffuser plates 10 are provided in the respective openings in the hollow chambers 8b, 8c to diffuse ultraviolet rays. At least a bottom surface 8f of the passage 8a, that is, the surface of the passage 8a which is opposite the opening 8a1 is black. This hinders light entering through the opening 8a1 from being reflected by an inner wall surface defining the passage space. At the same time, the light receiving element PD1 receives an ultraviolet ray emitted by the light source UV1 and transmitted through the yarn 1. The light receiving element PD2 receives an ultraviolet ray emitted by the light source UV1 and reflected by the yarn 1. The light receiving element PD2 receives an ultraviolet ray emitted by the light source UV2 and transmitted through the yarn 1. The light receiving element PD1 receives an ultraviolet ray emitted by the light source UV2 and reflected by the yarn 1. The light receiving element PD3 receives only a light emitted by the light sources UV1, UV2 and reflected by the yarn 1. That is, the light receiving element PD3 is used exclusively for reflected light to receive only reflected light. In this case, the diffuser plates 10 diffuse ultraviolet rays in all directions before the yarn 1 is irradiated with the rays. This makes it possible to increase the quantity of light received by the light receiving elements PD1, PD2, PD3.

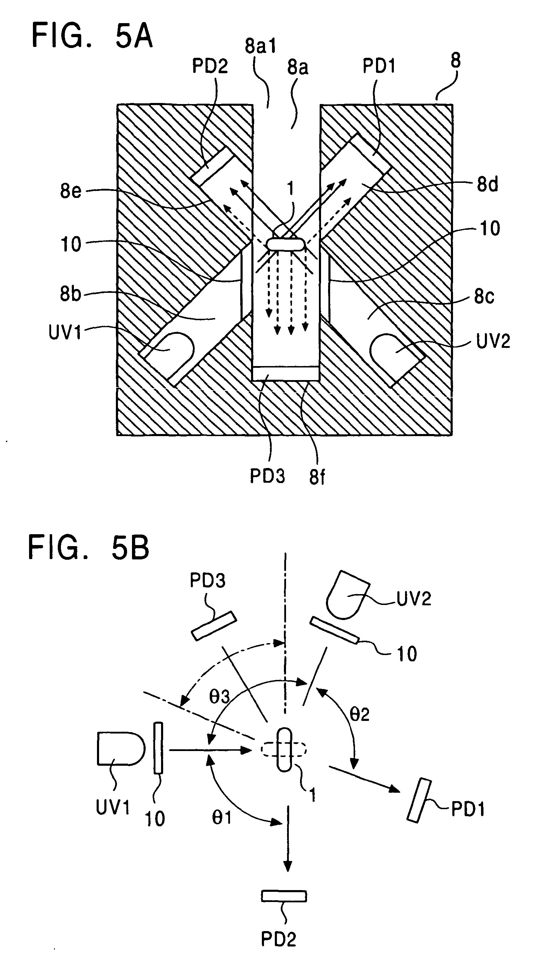

In the above specific example, the hollow chamber 8h is provided in which the reflected-light-excusive light receiving element PD3 receiving only reflected light is installed. As shown in Figure 5A, the light receiving element PD3 may be provided on the bottom surface 8f of the passage 8a. In Figure 5A, components corresponding to those in the specific example shown in Figure 4 are denoted by the same reference numerals. Their detailed description will be omitted.

In the arrangement shown in Figures 4 and 5A, on a plane orthogonal to the running yarn 1 (in a plan view), the light sources UV1, UV2 are arranged away from each other so as to form an angle of about 90 degres around the running yarn 1. The light receiving element PD1 is placed opposite the light source UV1 across the yarn 1. The light receiving element PD2 is placed opposite the light source UV2 across the yarn 1. The light receiving element PD3 is placed between the positions of the light source UV1 and the light source UV2, separated from each other by an angle of about 90 degrees.

This arrangement enables the light receiving element PD1 to receive both light from the light source UV1 which is transmitted through the yarn 1 and light from the light source UV2 which is reflected by the yarn 1 at 90 degrees. The light receiving element PD2 can receive both light from the light source UV2 which is transmitted through the yarn 1 and light from the light source UV1 which is reflected by the yarn 1 at 90 degrees. The light receiving element PD3 can receive light from the light sources UV1, UV2 which is reflected by the yarn 1 in a direction between the light receiving element PD1 and the light receiving element PD2. That is, if a polypropylene film is mixed into the yarn 1 to form flat foreign material, this defect can be detected over a wide angular range in which the flat portion appears.

However, with this arrangement, if for example, the foreign material is at a detected position with its flat sectional shape extending on the optical axis of the light source UV1, light from the light source UV2 is reflected toward the light source UV2. Accordingly, the light receiving element PD1 cannot detect the reflected light. Further, a light receiving surface of the light receiving element PD2 is opposite a surface of the flat yarn 1, so that even though the yarn 1 is irradiated with an ultraviolet ray from the light source UV1, the light is applied to a thin surface of the yarn 1. Consequently, the light cannot be reflected toward the light receiving elements PD2.

To avoid this drawback, the configuration described below is used.

As shown in Figure 5B, on a plane orthogonal to the running yarn 1, the angle of aperture θ3 between the light source UV1 and the light source UV2 around the running yarn 1 may be set between 90 degrees and 135 degrees. The angle of aperture θ1 between the light source UV1 and the light receiving element PD2, which receives a reflected light from the light source UV1 and a transmitted light from the light source UV2, may be set 90 degrees or smaller. The angle of aperture θ2 between the light source UV2 and the light receiving element PD1, which receives a reflected light from the light sourcee UV2 and a transmitted light from the light source UV1, may be set at 90 degrees or smaller. The light receiving elements PD2 and PD1 may be arranged closer to each other. The light receiving element PD3 may be arranged between the light source UV1 and the light source UV2, and within the angle (shown by the alternate long and short dash line) between the light receiving element PD1 and the light receiving element PD2 around the yarn 1. This arrangement and the placement of the diffuser plate 10 enable a flat yarn defect to be detected in all directions using a minimum number of light receiving elements.

For example, if a polypropylene film is mixed into the yarn 1, is flat, and lies at a detected position, for example, with its flat sectional shape extending on the optical axis of the light source UV1 as shown by the dotted line in Figure 5B, then the light receiving element PD2 receives that part of the ultraviolet light from the light source UV2 which is transmitted through the yarn 1. The light receiving element PD3 receives that part of the ultraviolet light from the light source UV2 which is reflected by the yarn 1. Further, if the polypropylene film is flat and lies at a detected position with its flat sectional shape extending on the optical axis of the light source UV2, then the light receiving element PD1 receives that part of the ultraviolet light from the light source UV1 which is transmitted through the yarn 1. The light receiving element PD3 receives that part of the ultraviolet light from the light source UV1 which is reflected by the yarn 1. It is thus possible to detect a flat portion that may appear at any position within an angle of 360 degrees.

In the above embodiment, the light sources generate ultraviolet rays. Accordingly, the quantity of light received varies very insignificantly depending on the thickness of a yarn containing cotton fibers as a material.

On the other hand, the quantity of light received varies significantly depending on the presence of homogeneous fibers of various colors or a heterogeneous white object such as a white polypropylene film. Suitably, a device can thus be obtained which can accurately detect a foreign-material defect simply by detecting the quantity of light reflected. In particular, if the yarn contains white cotton fibers as a material, when a cotton yarn is irradiated with light, the yarn reflects only a small quantity of light.

Further, the quantity of light reflected is not substantially affected by the yarn thickness. However, the cotton yarn reflects a large quantity of light when foreign material is mixed into the yarn. Consequently, an accurate detecting device for foreign material can be obtained which can reliably detect the mixture of homogeneous fibers of different colors or, in particular, a heterogeneous white object such as a white polypropylene film. However, since the exclusive light receiving element is separately provided which diffuses light applied to the yarn and which receives light reflected by the yarn, the light source may generate another visible ray or an infrared ray. Alternatively, such a light source may be combined with a light source that generates an ultraviolet ray.

In the above description of the embodiment, the white cotton yarn is used as a material for a spun yarn. However, the present invention is not limited to this aspect. The present invention is applicable to the case where a spun yarn is generated by mixing, for example, polyester, a kind of synthetic fiber, which has been cut into equal lengths, with cotton (both are white).

## Claims

1. A detecting device for foreign material in a yarn (1), comprising a plurality of floodlighting means (UV1, UV2) for irradiating the running yarn (1) with light from different directions and three light receiving means (PD1, PD2, PD3) for receiving that part of the light applied by said floodlighting means (UV1, UV2) which is reflected by said yarn (1), and in that foreign material mixed into said yarn (1) is detected on the basis of the quantity of reflected light received by said light receiving means (PD1, PD2, PD3), wherein on a plane orthogonal to the running yarn (1),
• two of the floodlighting means (UV1, UV2) are arranged away from each other so as to form an angle (θ3) of less than 180 degrees, and
• one of the light receiving means (PD3) is placed between said spaced positions of the two floodlighting means (UV1, UV2),
**characterized in that**
• the device includes a time division circuit (6) for alternately driving said floodlighting means (UV1, UV2) and adders (4a, 4b) in order to obtain
a first signal (R) as an addition of the signals of the two light receiving means (PD1, PD2) depending on those parts of the light applied by said floodlighting means (UV1, UV2) which are reflected by said yarn (1),
a second signal (F) as an addition of the signals of the two light receiving means (PD1, PD2) depending on those parts of the light applied by said floodlighting means (UV1, UV2) transmitted through said yarn (1) and
o third signals (C) of said one light receiving means (PD3) depending on those parts of the light applied by said floodlighting means (UV1, UV2) which are reflected by said yarn (1).

2. A detecting device for foreign material in a yarn (1) according to claim 1,
**characterized in that**
• on a plane orthogonal to the running yarn (1), the remaining two of the light receiving means (PD1, PD2) are arranged opposite said respective floodlighting means (UV1, UV2) across the yarn (1) to receive those parts of the light applied by said floodlighting means (UV1, UV2) which are reflected by and transmitted through said yarn (1), and
• said one of the light receiving means (PD3) placed between the positions of said spaced floodlighting means (UV1, UV2) receives only that part of the light applied by said floodlighting means (UV1, UV2) which is reflected by said yarn (1).

3. A detecting device for foreign material in a yarn (1) according to claim 1 or claim 2,
**characterized in that**
the running yarn (1) is irradiated with ultraviolet light, and foreign material mixed into said yarn (1) is detected on the basis of the quantity of received light reflected by said yarn (1).

4. A detecting device for foreign material in a yarn (1) according to one of the preceeding claims,
**characterized in that**
on a plane orthogonal to the running yarn (1), two of the floodlighting means (UV1, UV2) are arranged away from each other so as to form an angle (θ3) with respect to the position of the yarn (1) between 90 degrees and 135 degrees and one of the light receiving means (PD3) is placed between the positions of said spaced floodlighting means (UV1, UV2), **in that** the remaining two light receiving means (PD1, PD2) are arranged out of said angle between 90 degrees and 135 degrees and away from each of said floodlighting means (UV1, UV2) so as to form an angle (θ2, θ1) of 90 degrees or less with said floodlighting means (UV1, UV2), and **in that** one of said light receiving means (PD3) is placed within the opposite angle of the angle formed by said remaining two light receiving means (PD1, PD2) around said yarn.

## Patentansprüche

1. Vorrichtung zum Detektieren von Fremdmaterial in einem Faden (1) umfassend mehrere Flutlichtmittel (UV1, UV2) zur Beleuchtung des laufenden Fadens (1) mit Licht aus verschiedenen Richtungen und drei Lichtempfangsmitteln (PD1, PD2, PD3) zum Empfangen desjenigen Teils des von den Flutlichtmitteln (UV1, UV2) zugeführten Lichts, der von dem Faden (1) reflektiert wird, wobei in den Faden (1) eingemischtes Fremdmaterial auf Basis der Menge des von den Lichtempfangsmitteln (PD1, PD2, PD3) empfangenen reflektierten Lichts detektiert wird, wobei in einer zum laufenden Faden (1) senkrechten Ebene
- zwei der Flutlichtmittel (UV1, UV2) voneinander beabstandet angeordnet sind, wobei sie einen Winkel (θ 3) von weniger als 180° bilden, und
- eines der Lichtempfangsmittel (PD3) zwischen den beabstandeten Positionen der beiden Flutlichtmittel (UV1, UV2) angeordnet ist,
**dadurch gekennzeichnet, dass**
- die Vorrichtung eine Zeitteilerschaltung (6) zum abwechselnden Betrieb der Flutlichtmittel (UV1, UV2) sowie Addiereinrichtungen (4a, 4b) umfasst, um
- ein erstes Signal (R) als Addition der Signale der beiden Lichtempfangsmittel (PD1, PD2) zu erhalten, die auf denjenigen Teilen des von den Flutlichtmitteln (UV1, UV2) zugeführten Lichts beruhen, die von dem Faden (1) reflektiert werden,
- ein zweites Signal (F) als Addition der Signale der beiden Lichtempfangsmittel (PD1, PD2) zu erhalten, die auf denjenigen Teilen des von den Flutlichtmitteln (UV1, UV2) zugeführten Lichts beruhen, die durch den Faden (1) transmittiert werden, und
- dritte Signale (C) des einen Lichtempfangsmittels (PD3) zu erhalten, die auf denjenigen Teilen des von den Flutlichtmitteln (UV1, UV2) zugeführten Lichts beruhen, die von dem Faden (1) reflektiert werden.

2. Vorrichtung zum Detektieren von Fremdmaterial in einem Faden (1) gemäß Anspruch 1,
**dadurch gekennzeichnet, dass**
- in einer Ebene senkrecht zum laufenden Faden (1) die verbleibenden beiden Lichtempfangsmittel (PD1, PD2) gegenüberliegend der jeweiligen Flutlichtmittel (UV1, UV2) auf der anderen Seite des Fadens (1) angeordnet sind, um diejenigen Teile des von den Flutlichtmitteln (UV1, UV2) zugeführten Lichts zu empfangen, die von dem Faden (1) reflektiert und durch diesen transmittiert werden, und
- das Lichtempfangsmittel (PD3), das zwischen den Positionen der voneinander beabstandeten Flutlichtmittel (UV1, UV2) angeordnet ist, nur denjenigen Teil des von den Flutlichtmitteln (UV1, UV2) zugeführten Lichts empfängt, der von dem Faden (1) reflektiert wird.

3. Vorrichtung zum Detektieren von Fremdmaterial in einem Faden (1) gemäß Anspruch 1 oder 2,
**dadurch gekennzeichnet, dass**
der laufende Faden (1) mit UV-Licht bestrahlt wird und in den Faden (1) eingemischtes Fremdmaterial auf Basis der Menge an empfangenen Lichts, das von dem Faden (1) reflektiert wird, detektiert wird.

4. Vorrichtung zum Detektieren von Fremdmaterial in einem Faden (1) gemäß einem der vorangehenden Ansprüche,
**dadurch gekennzeichnet, dass**
in einer Ebene senkrecht zum laufenden Faden (1) zwei der Flutlichtmittel (UV1, UV2) voneinander beabstandet angeordnet sind, so dass sie einen Winkel (θ 3) bezüglich der Position des Fadens (1) bilden, der zwischen 90° und 135° liegt, wobei eines der Lichtempfangsmittel (PD3) zwischen den Positionen der voneinander beabstandeten Flutlichtmittel (UV1, UV2) angeordnet ist und die beiden verbleibenden Lichtempfangsmittel (PD1, PD2) außerhalb des Winkels zwischen 90° und 135° und von jedem der beiden Flutlichtmittel (UV1, UV2) beabstandet angeordnet sind, um mit den Flutlichtmitteln (UV1, UV2) einen Winkel (θ2, θ1) von 90° oder weniger zu bilden, und wobei das eine Lichtempfangsmittel (PD3) innerhalb des Gegenwinkels des von den beiden verbleibenden Lichtempfangsmittel (PD1, PD2) um den Faden gebildeten Winkels liegt.

## Revendications

1. Dispositif pour la détection de matières étrangères dans un fil (1), comprenant
une pluralité de moyens d'illumination (UV1, UV2) pour l'irradiation du fil en mouvement (1) avec de la lumière de différentes directions et trois moyens de réception de lumière (PD1, PD2, PD3) pour recevoir cette partie de la lumière appliquée par lesdits moyens d'éclairage (UV1, UV2) qui est réfléchie par ledit fil (1), et en ce que des matières étrangères mélangées dans ledit fil (1) sont détectées sur la base de la quantité de lumière réfléchie reçue par lesdits moyens de réception de lumière (PD1, PD2, PD3), où sur un plan orthogonal au fil en mouvement (1),
• deux des moyens d'éclairage (UV1, UV2) sont agencés l'un au loin de l'autre de manière à former un angle (θ 3) inférieur à 180 degrés, et
• un des moyens de réception de lumière (PD3) est placé entre lesdites positions espacées des deux moyens d'éclairage (UV1, UV2),
**caractérisé en ce que**
• le dispositif comprend un circuit de division de temps (6) pour entraîner alternativement lesdits moyens d'éclairage (UV1, UV2) et des additionneurs (4a, 4b) pour obtenir un premier signal (R) comme une addition des signaux des deux moyens de réception de lumière (PD1, PD2) en fonction de ces parties de la lumière appliquée par lesdits moyens d'éclairage (UV1, UV2) qui sont réfléchies par ledit fil (1),
un second signal (F) comme une addition des signaux des deux moyens de réception de lumière (PD1, PD2) dépendant de ces parties de la lumière appliquées par lesdits moyens d'éclairage (UV1, UV2) transmises à travers ledit fil (1) et
des troisièmes signaux (C) d'un moyen de réception de lumière précité (PD3) dépendant de ces parties de la lumière appliquée par lesdits moyens d'éclairage (UV1, UV2) qui sont réfléchies par ledit fil (1).

2. Dispositif pour la détection de matières étrangères dans un fil (1) selon la revendication 1,
**caractérisé en ce que**
• sur un plan orthogonal au fil en mouvement (1), les deux moyens restants des moyens de réception de lumière (PD1, PD2) sont agencés d'une manière opposée auxdits moyens d'éclairage respectifs (UV1, UV2) sur le fil (1) pour recevoir ces parties de la lumière appliquées par lesdits moyens d'éclairage (UV1, UV2) qui sont réfléchies par et transmises à travers ledit fil (1), et
• le moyen de réception de lumière précité (PD3) placé entre les positions desdits moyens d'éclairage espacés (UV1, UV2) reçoit seulement cette partie de la lumière appliquée par lesdits moyens d'éclairage (UV1, UV2) qui est réfléchie par ledit fil (1).

3. Dispositif pour la détection de matières étrangères dans un fil (1) selon la revendication 1 ou la revendication 2,
**caractérisé en ce que** le fil en mouvement (1) est irradié avec de la lumière ultraviolette, et des matières étrangères mélangées dans ledit fil(1) sont détectées sur la base de la quantité de la lumière reçue réfléchie par ledit fil (1).

4. Dispositif pour la détection de matières étrangères dans un fil (1) selon l'une des revendications précédentes,
**caractérisé en ce que**
sur un plan orthogonal au fil en mouvement (1), deux des moyens d'éclairage (UV1, UV2) sont agencés l'un au loin de l'autre de manière à former un angle (θ 3) par rapport à la position du fil entre 90 degrés et 135 degrés, et un des moyens de réception de lumière (PD3) est placé entre les positions desdits moyens d'éclairage espacés (UV1, UV2), **en ce que** les deux moyens de réception de lumière restants (PD1, PD2) sont agencés en dehors dudit angle entre 90 degrés et 135 degrés et au loin de chacun desdits moyens d'éclairage (UV1, UV2) de manière à former un angle (θ 2, θ 1) de 90 degrés ou moins relativement auxdits moyens d'éclairage (UV1, UV2), et **en ce que** l'un desdits moyens de réception de lumière (PD3) est placé dans l'angle opposé à l'angle formé par lesdits deux moyens de réception de lumière restants (PD1, PD2) autour dudit fil.
